# EUROPEAN PATENT APPLICATION

(11) **EP 1 547 520 A1**
(43) Date of publication of application: **29.06.2005**
(21) Application number: 04256205.8
(22) Date of filing: 07.10.2004
(51) Int. Cl.: A61B 5/0452, A61B 5/04, A61N 1/37

(54) **Electrogram signal filtering in systems for detecting ischemia**

(30) Priority: 22.12.2003 US 741141
(71) Applicant: Angel Medical Systems, Inc, Fair Haven, New Jersey 07704 (US)
(72) Inventor: Fischell, David R., Fair Haven, New Jersey 07704 (US); Fischell, Tim A., Kalamazoo MI 49008 (US)
(74) Representative: Harris, Ian Richard

(57) **Abstract**

The present system utilizes optimized high pass or band pass filtering to provide accurate detection of changes in the ST segment of an electrogram as detected by an implanted cardiosaver system, which ST segment changes are indicative of coronary ischemia. To accurately detect changes in the ST segment of the electrogram, the cardiosaver system uses either analog or digital high pass filtering having an electrogram voltage reduction of 6 dB at some frequency between 0.05 Hz and 1.0 Hz with less than 6 dB of attenuation at frequencies above 1.0 Hz and more than 6 dB of attenuation for frequencies below 0.05 Hz. The optimized filter would include attenuation of electric power generation frequencies of either 50 Hz or 60 Hz to reduce electromagnetic interference. The system also includes both implanted and external portions that provide a patient alarm if coronary ischemia is sensed.

## Description

### FIELD OF USE

This invention is in the field of implantable medical device systems that monitor a patient's cardiovascular condition.

### BACKGROUND OF THE INVENTION

Heart disease is the leading cause of death in the United States. A heart attack (also known as an acute myocardial infarction (AMI)) typically results from a thrombus (i.e., a blood clot) that obstructs blood flow in one or more coronary arteries. AMI is a common and life-threatening complication of coronary artery disease. Coronary ischemia is caused by an insufficiency of oxygen to the heart muscle. Ischemia is typically provoked by physical activity or other causes of increased heart rate when one or more of the coronary arteries are narrowed by atherosclerosis. Patients will often (but not always) experience chest discomfort (angina) when the heart muscle is experiencing ischemia. Those with coronary atherosclerosis are at higher risk for AMI if the plaque becomes further obstructed by thrombus.

The current treatment for a coronary artery narrowing (a stenosis) is the insertion of a drug eluting stent such as the Cypher™ sirolimus-eluting stent from Cordis Corporation or the Taxus™ paclitaxel-eluting stent from the Boston Scientific Corporation. The insertion of a stent into a stenosed coronary artery is a reliable medical treatment to eliminate or reduce coronary ischemia and to prevent the complete blockage of a coronary artery, which blockage can result in an AMI.

Acute myocardial infarction and ischemia may be detected from a patient's electrocardiogram (ECG) by noting an ST segment shift (i.e., voltage change). However, without knowing the patient's normal ECG pattern, detection from a standard 12 lead ECG can be unreliable.

Fischell et al in U.S. Patents Numbered 6,112,116, 6,272,379 and 6,609,023 describe implantable systems and algorithms for detecting the onset of acute myocardial infarction and providing both patient alerting and treatment. The Fischell et al patents describe how the electrical signal from inside the body (which is called an "electrogram") can be used to determine various states of myocardial ischemia.

The Reveal™ subcutaneous loop Holter monitor sold by Medtronic, Inc., uses two case electrodes spaced about 3 inches apart to record electrogram information. Recording can be triggered automatically when arrhythmias are detected or upon patient initiation using an external device. The Reveal is designed to record electrogram data and does not include the signal processing capability to track changes in the heart signal over an extended period of time. The Reveal also does not have the capability to measure or alert the patient if there is an ST segment shift. In fact, the Reveal's high pass filtering and electrode spacing preclude accurate detection of changes in the low frequency aspects of the heart's electrical signal such as the ST segment of the electrogram. Those low frequency aspects of the heart's electrical signal are required for the accurate detection of ST segment shift that is a measure of coronary ischemia.

While pacemakers and Implantable Cardioverter Defibrillators (ICDs) collect electrogram data and track the numbers of beats paced or not paced, they do not currently detect ST segment changes. In fact, the filters typically used in pacemakers and ICDs have a high pass filter setting that precludes the accurate detection of ST segment changes. Specifically a high pass filter setting that has more than 6 dB of attenuation at frequencies at or above 1 Hz will mask any changes in ST segment shifts. Any significant attenuation of electrogram signal voltage at frequencies between 1 and 2 Hz is less than ideal for the accurate detection of changes in the ST segment of the electrogram. The inability to detect changes in the amplitude of the ST segment voltage severely compromises the ability of an implanted device to detect coronary ischemia.

Although some ICDs have an unfiltered channel for electrogram recording, an unfiltered electrogram signal is less than ideal for the accurate determination of a shift in the electrogram's ST segment. This is because it is highly desirable to have a high pass filter with at least 6 dB of attenuation at a frequency of approximately 0.1 Hz to prevent excessive drift in the electrogram signal, which drift makes it difficult to accurately detect ST segment shift.

The term "medical practitioner" shall be used herein to mean any person who might be involved in the medical treatment of a patient. Such a medical practitioner would include, but is not limited to, a medical doctor (e.g., a general practice physician, an internist or a cardiologist), a medical technician, a paramedic, a nurse or an electrogram analyst. Although the masculine pronouns "he" and "his" are used herein, it should be understood that the patient, physician or medical practitioner could be a man or a woman. A "cardiac event" includes an acute myocardial infarction, ischemia caused by effort (such as exercise) and/or an elevated heart rate, bradycardia, tachycardia or an arrhythmia such as atrial fibrillation, atrial flutter, ventricular fibrillation, and premature ventricular or atrial contractions (PVCs or PACs).

It is generally understood that the term "electrocardiogram" is defined as the heart electrical signals sensed by means of skin surface electrodes that are placed in a position to indicate the heart's electrical activity (depolarization and repolarization). An electrocardiogram segment refers to a portion of electrocardiogram signal that extends for either a specific length of time, such as 10 seconds, or a specific number of heart beats, such as 10 beats. As used herein, the PQ segment of a patient's electrocardiogram or electrogram is the typically straight segment of a beat of an electrocardiogram or electrogram that occurs just before the R wave and the ST segment is a typically straight segment that occurs just after the R wave.

Although often described as an electrocardiogram (ECG), the electrical signal from the heart as measured from electrodes within the body is properly termed an "electrogram". As defined herein, the term "electrogram" is the heart's electrical signal voltage as sensed from one or more implanted electrode(s) that are placed in a position to indicate the heart's electrical activity (depolarization and repolarization). An electrogram segment refers to a portion of the electrogram signal for either a specific length of time, such as 10 seconds, or a specific number of heart beats, such as 10 beats. For the purposes of this specification, the terms "detection" and "identification" of a cardiac event have the same meaning. A beat is defined as a subsegment of an electrogram or electrocardiogram segment containing exactly one R wave.

A heart signal parameter is defined to be a measured or calculated value created during the processing of one or more beats of the electrogram (or electrocardiogram). Heart signal parameters include median or average ST segment voltage, ST deviation which is ST segment average voltage minus PQ segment average voltage, ST shift which is ST deviation compared to a baseline average ST deviation, ST elevation which is a positive change of the ST segment voltage, ST depression which is a negative change of the ST segment voltage, T wave peak height, T wave average value, T wave deviation, QRS complex width, number of PVCs per unit time, heart rate and R-R interval.

### SUMMARY OF THE INVENTION

Various aspects of the invention are set out in the accompanying claims.
An example of an entire system for the detection of coronary ischemia described herein is referred to as a "Guardian" system. The Guardian system detects coronary ischemia using an implanted sub-system called a "cardiosaver system" which is designed to detect coronary ischemia by the measurements of a change in the ST segment voltage including ST depression or ST elevation. The most sever form of coronary ischemia is from an AMI which is essentially a complete blockage of a coronary artery. A change in the ST segment indicative of ischemia can be detected by a cardiosaver through measurement of just the ST segment voltage (as compared to a zero voltage level), by measurement of ST deviation (ST segment voltage minus PQ segment voltage), and/or by computation of ST shift (current beat ST deviation as compared to a previously collected average baseline ST deviation value).

The cardiosaver system includes electrodes placed to advantageously sense electrical signals from the heart that is the electrogram. The electrodes can be placed within the heart and/or subcutaneously. The implanted portion of the Guardian system is the cardiosaver system as described by Fischell et al in U.S. Patents Numbered 6,112,116, 6,272,379 and 6,609,023, each of these patents being incorporated herein by reference. The Guardian system also includes external equipment that can include a physician's programmer and an external alarm device also described in the Fischell et al patents.

An example embodiment of the invention can provide a cardiosaver system that utilizes optimized high pass or band pass filtering to provide accurate detection of ST segment shifts. Either analog or digital high pass filtering having a voltage magnitude reduction of less than 6 dB at 1 Hz can be used. Using high pass filtering of greater than 6dB at 1Hz high pass filter having a voltage magnitude reduction of 6 dB at 0.5 Hz there is some masking of the ST segment shift. A preferred high pass filter has less than 6 dB voltage magnitude reduction at 0.5 Hz.

An example of high pass filtering can have voltage magnitude reduction greater than 6 dB at frequencies at or below 0.05 Hz. Without such high pass filtering, there is potential for d-c signal drift which can cause the ST segment level measurement to drift up or down in voltage over time. The ST shift detection algorithm described by Fischell et al in U.S. Patent No. 6,609,023 is designed to work even in the presence of some d-c drift. The Fischell algorithm does this by looking for a shift in the ST deviation (average ST segment voltage minus average PQ segment voltage) instead of using just the magnitude of the ST segment voltage. However, even when ST deviation is used for ST segment shift detection, it is still better to reduce d-c signal drift by the use of a very low frequency high pass filter. A preferred embodiment of the present invention cardiosaver system uses a high pass filter with an electrogram signal voltage magnitude reduction of greater than 6 dB at or below a frequency of 0.1 Hz.

Another aspect of the present invention envisions having no high pass filtering (i.e., the electrodes are direct coupled through the amplifier and signal processor) with ischemia being detected by the measurement of a change in ST segment deviation as defined herein. Although direct coupling would allow d-c drift, the measurement of the difference in voltage between the ST segment and the PQ segment around the same R wave tends to minimize the measurement error produced by d-c drift. Even if no high pass filtering is used, some low pass filtering to avoid interference from power generation frequencies can be envisioned to reduce or eliminate any interference from that source of interference. Thus, a low pass filter having at least 6 dB of attenuation at 60 HZ compared to the electrogram signal magnitude at 25 Hz is envisioned. Still further, an attenuation of greater than 6 dB at 50 Hz compared to the electrogram signal can magnitude at 25 Hz. can also be envisioned.

Since the ST segment is a relatively low frequency signal, there is no need to include signals at frequencies above 50 Hz. A 60 Hz low pass filter (i.e., a filter that has at least 6 dB of attenuation at 60 Hz) will essentially eliminate any stray interference from a-c power signals for countries using 60 Hz power generation. A 50 Hz low pass filter (i.e., a filter that has at least 6 dB of attenuation at 50 Hz) will essentially eliminate any stray interference from a-c power signals for countries using 50 Hz power generation. The 50 Hz low pass filter is preferred as it would also work in countries with 60 Hz power. The preferred embodiment of the present invention utilizes a band pass filter that attenuates by at least 6 dB those frequency components of the electrogram that are lower than 0.25 Hz or higher than 25 Hz. Optimally, the band pass filter in the cardiosaver of the cardiosaver system provides at least 6 dB of electrogram signal voltage attenuation for all frequency components at or below 0.1 Hz and at least 6 dB of attenuation for all frequency components at or above 50 Hz.

A preferred embodiment cardiosaver system can also include a patient alerting capability that is also described in the Fischell et al patents. This patient alerting capability is designed to warn the patient if an ST segment shift indicative of ischemia has been detected.

Another embodiment of the present invention can provide an implanted ischemia detection device that also includes pacemaker circuitry to pace the patient's heart as needed. Still another embodiment can provide an implanted ischemia detection device that includes ICD circuitry to defibrillate the patient's heart as needed. Yet another embodiment can provide an implanted ischemia detection device that includes a combination of pacemaker and ICD circuitry. Each of these pacemaker and/or ICD devices can include the previously described electrogram signal filtering (or d-c coupling with ischemia detection by the measurement of ST deviation) as described herein.

Thus, an example of this invention can provide a cardiosaver system that can process the electrical signal from a patient's heart where the processing includes a high pass filter having a voltage magnitude reduction of less than 6 dB at 1 Hz compared to the voltage magnitude at 3 Hz.

An example of this invention can provide a cardiosaver system that can process the electrical signal from a patient's heart where the electrical signal processing includes a high pass filter having a voltage magnitude reduction of less than 6 dB at 0.5 Hz as compared to the voltage magnitude at 1.5 Hz.

An example of the invention can provide a cardiosaver system that can process the electrical signal from a patient's heart where the processing includes a high pass filter having a voltage magnitude reduction of greater than 6 dB at 0.1 Hz compared to the voltage magnitude at 0.3 Hz.

An example of the invention can provide a cardiosaver system that can process the electrical signal from a patient's heart where the processing includes a 60 Hz low pass filter.

An example of the invention can provide a cardiosaver system that can process the electrical signal from a patient's heart where the processing includes a 50 Hz low pass filter.

An example of the invention can provide a pacemaker with ischemia detection where the processing of electrical signals from the patient's heart includes a high pass filter having a voltage magnitude reduction of less than 6 dB at 1 Hz compared to the voltage magnitude at 3 Hz.

An example of the invention can provide a pacemaker with ischemia detection where the processing of electrical signals from the patient's heart includes a high pass filter having a voltage magnitude reduction of less than 6 dB at 0.5 Hz as compared to the voltage magnitude at 1.5 Hz.

An example of the invention can provide a pacemaker with ischemia detection where the processing of electrical signals from the patient's heart includes a 60 Hz low pass filter.

An example of the invention can provide a pacemaker with ischemia detection where the processing of electrical signals from the patient's heart includes a 50 Hz low pass filter.

An example of the invention can provide an ICD with ischemia detection where the processing of electrical signals from the patient's heart includes a high pass filter having a voltage magnitude reduction of less than 6 dB at 1 Hz compared to the voltage magnitude at 3 Hz.

An example of the invention can provide an ICD with ischemia detection where the processing of electrical signals from the patient's heart includes a high pass filter having a voltage magnitude reduction of less than 6 dB at 0.5 Hz compared to the voltage magnitude at 1.5 Hz.

An example of the invention can provide an ICD with ischemia detection where the processing of electrical signals from the patient's heart includes a 60 Hz low pass filter.

An example of the invention can provide an ICD with ischemia detection where the processing of electrical signals from the patient's heart includes a 50 Hz low pass filter.

An example of the invention can provide a cardiosaver system with ischemia detection where the processing of electrogram signal voltage from the patient's heart includes a high pass filter having a voltage magnitude reduction greater that 6 dB at a frequency that lies between 0.05 Hz and 1.0 Hz.

An example of the invention can provide a cardiosaver system with ischemia detection where the processing of electrogram signal voltage from the patient's heart includes a high pass filter having a voltage magnitude reduction greater that 6 dB at a frequency that lies between 0.1 Hz and 0.5 Hz.

These and other examples and advantages of this invention will become apparent to a person of ordinary skill in this art upon reading of the detailed description of this invention including the associated drawings as presented herein.

### BRIEF DESCRIPTION OF THE DRAWINGS

FIG. 1 illustrates a Guardian system for the detection of a cardiac event such as an ST segment shift indicative of coronary ischemia and for warning the patient that a cardiac event is occurring.
FIG. 2 is a block diagram of the implanted cardiosaver system.
FIG. 3A illustrates the effect of a 0.1 Hz high pass filter on the ST segment of the electrogram from a patient's heart.
FIG. 3B illustrates the effect of a 0.5 Hz high pass filter on the ST segment of the electrogram from a patient's heart.
FIG. 3C illustrates the effect of a 1 Hz high pass filter on the ST segment of the electrogram from a patient's heart.
FIG. 3D illustrates the effect of a 2 Hz high pass filter on the ST segment of the electrogram from a patient's heart.
FIG. 4 shows the attenuation as a function of frequency for the 1 Hz high pass filter used to process the data of FIG. 3C.
FIG. 5 shows the attenuation as a function of frequency response for the 2 Hz high pass filter used to process the data of FIG. 3D.
FIG. 6A illustrates the effect of a 0.1 Hz high pass filter on the d-c drift of the electrogram from a patient's heart.
FIG. 6B illustrates the effect of a 0.25 Hz high pass filter on the d-c drift of the electrogram from a patient's heart.
FIG. 7 illustrates the range of electrogram frequencies that should be attenuated for accurate detection of ST segment shifts from an implanted cardiosaver system.

### DETAILED DESCRIPTION OF THE INVENTION

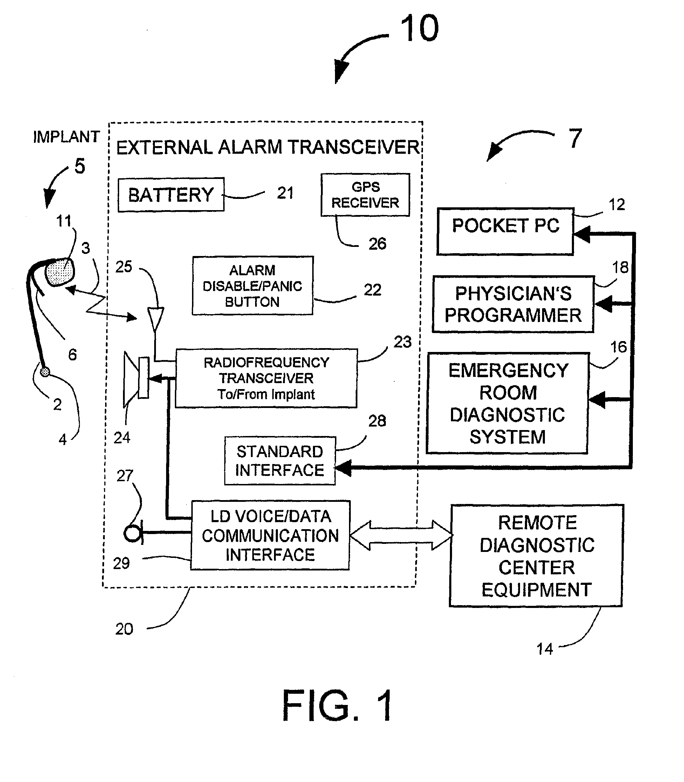

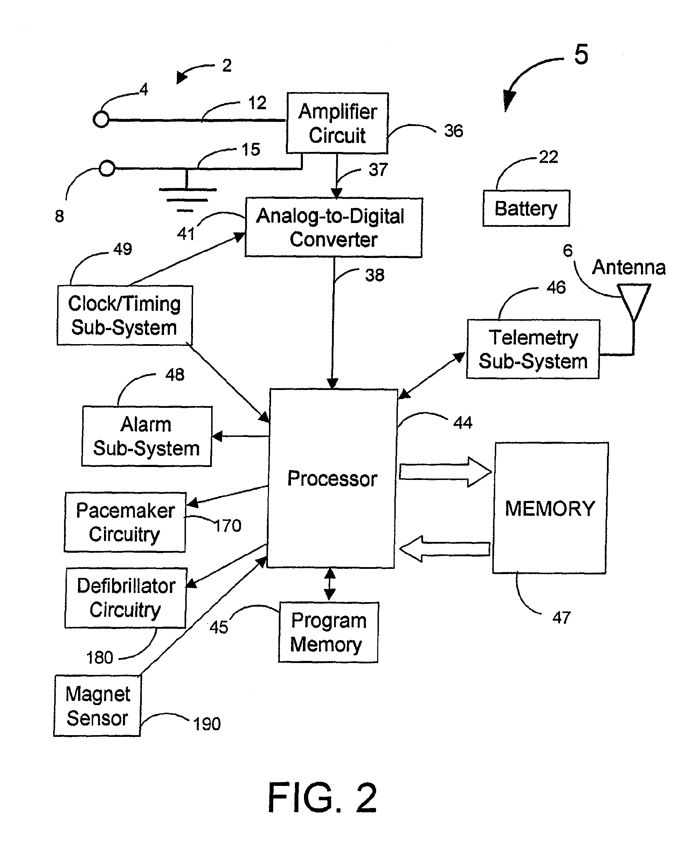

FIG. 1 illustrates one embodiment of the Guardian system 10 consisting of an implanted cardiosaver system 5 and external equipment 7. The cardiosaver system 5 includes a cardiosaver 11, an antenna 6 and an electrode 4 that is part of a lead 2. The cardiosaver 11 includes electronic circuitry that can detect a cardiac event such as an acute myocardial infarction or arrhythmia and can warn the patient when a cardiac event occurs. The cardiosaver 11 can store the patient's electrogram for later readout and can send and receive wireless signals 3 to and from the external equipment 7 via the implanted antenna 6 and the external antenna 25. The functioning of the cardiosaver system 5 will be explained in greater detail with the assistance of FIG. 2.

The cardiosaver system 5 has at least one lead 2 with at least one electrode 4. In fact, the cardiosaver system 5 could utilize as few as one lead or as many as three and each lead could have as few as one electrode or as many as eight electrodes. The lead 2 in FIG. 1 would advantageously be placed subcutaneously or through the patient's vascular system with the electrode 4 being placed into the apex of the right ventricle. For example, the lead 2 could be placed in the right ventricle or right atrium or the superior vena cava similar to the placement of leads for pacemakers and ICDs. The metal case of the cardiosaver 11 could serve as an indifferent electrode with the electrode 4 being the active electrode. Alternately, the lead 2 in FIG. 1 could be placed through the patient's vascular system with the electrode 4 being placed into the apex of the left ventricle.

The lead 2 could advantageously be placed subcutaneously at any location where the electrode 4 would provide a good electrogram signal indicative of the electrical activity of the heart. Again for the lead 2, the case of the cardiosaver 11 of the cardiosaver system 5 could be an indifferent electrode and the electrode 4 would be the active electrode. Although the Guardian system 10 described herein can readily operate with only two electrodes, or one electrode and the case of the cardiosaver being the other electrode, it is envisioned that multiple electrodes used in monopolar or bipolar configurations could be used.

FIG. 1 also shows the external equipment 7 that consists of an external alarm transceiver 20, a physician's programmer 18, a pocket PC 12, an emergency room diagnostic system 16 and the equipment 14 in a remote diagnostic center. The external equipment 7 provides the means to interact with the cardiosaver system 5. These interactions include programming the cardiosaver 11, retrieving data collected by the cardiosaver system 5 and handling alarms generated by the cardiosaver 11. It should be understood that the cardiosaver system 5 could operate with some but not all of the external equipment 7.

The external alarm transceiver 20 includes a battery 21, an alarm disable/panic button 22, a radio frequency transceiver 23, a microphone 27, an alarm-speaker 24 an antenna 25, a GPS satellite receiver 26 and a standard interface 28 for providing wired or wireless communication with the pocket PC 12, remote diagnostic center equipment 14, emergency room diagnostic system 16 or physician's programmer 18. A long distance voice/data communications interface 29 provides connectivity to the remote diagnostic center equipment 14 through voice and data telecommunications networks. For example, the microphone 27 and speaker 24 could be used for wired or wireless telephone calls to and from a medical practitioner at the remote diagnostic center. A built-in modem as part of the interface 29 would allow data to be transmitted to and from the remote diagnostic center equipment 14 over a voice connection. Alternately, a data communications capability of the interface 29 could allow data to be sent or received through a wired or wireless data network. The external alarm transceiver 20 may be a separate unit that can be carried by the patient and used by the patient's physician as the data interface to the cardiosaver system 5 or it may also be built into the pocket PC 12, physician's programmer 18 or emergency room diagnostic system 16.

The pocket PC also described by Fischell et al in U.S. Patent No. 6,609,023 can provide the patient or physician the ability to check the status of the cardiosaver 11 and display a limited set of electrogram data uploaded from the cardiosaver 11.

The emergency room diagnostic system 16 is a more sophisticated system that can upload and display any of the data stored within the cardiosaver 11 and would, in its preferred embodiment, use a touch screen display to facilitate triage of patients arriving in an emergency room who have the cardiosaver system 5. This should greatly reduce the time from arrival at the emergency room until treatment for cardiosaver system patients having a cardiac event.

The purpose of the physician's programmer 18 shown in FIG. 1 is to set and/or change the operating parameters of the implanted cardiosaver system 5 and to read out data stored in the memory of the cardiosaver 11 such as stored electrogram segments as described by Fischell et al in U.S. Patent No. 6,609,023.

The external alarm transceiver 20 would typically be a pager-sized device that the patient would carry on his person or keep in close proximity. If a cardiac event is detected by the cardiosaver system 5, an alarm message is sent by a wireless signal 3 to the alarm transceiver 20 via the antennas 6 and 25. When the alarm is received by the alarm transceiver 20, a patient alerting sound is played through the loudspeaker 24 to warn the patient that a cardiac event has occurred. Examples of such sounds include a periodic buzzing, a sequence of tones and/or a speech message that instructs the patient as to what actions should be taken. Furthermore, the alarm transceiver 20 can, depending upon the nature of the signal 3, send an outgoing message to the remote diagnostic center equipment 14 to alert medical practitioners that a cardiosaver system alarm has occurred. The medical practitioners can then utilize the voice communications capabilities of the remote diagnostic center equipment 14 to call back the patient similar to the call that occurs with car drivers through the ONSTAR service when their car's air bags deploy in an accident. The optional GPS receiver 26 would allow the data sent to the remote diagnostic center equipment 14 to include patient location to facilitate the summoning of emergency medical services.

The alarm disable/panic button 22 will turn off both the internal alarm of the implant 5 and the sound being emitted from the loudspeaker 24. If no alarm is occurring, then pressing the alarm disable/panic button 22 will place a voice and/or data call to the remote diagnostic center similar to the call that is placed when the ONSTAR button is pressed in a car equipped to access the ONSTAR service. GPS information and a subset of patient electrogram data may be sent as well to the medical practitioners at the remote diagnostic center. The remotely located medical practitioner could then analyze the electrogram data and call the patient back to offer advice as to whether there is an emergency situation or the situation could be routinely handled by the patient's personal physician at some later time.

FIG. 2 is a block diagram of the cardiosaver system 5. The lead 2 includes the electrode 4 and the wire 12.The wire 12 connects the electrode 4 to the amplifier circuit 36 that is also connected by the wire 15 to the electrode 8 which could be a separate electrode or it could be the cardiosaver case acting as an indifferent electrode. The amplified electrogram signals 37 from the amplifier circuit 36 are converted to digital signals 38 by the analog-to-digital converter 41. The digital electrogram signals 38 are then sent to the electrical signal processor 44. The processor 44 in conjunction with the memory 47 can process the digital signals 38 according to the programming instructions stored in the program memory 45. This programming (i.e. software) enables the cardiosaver system 5 to detect the occurrence of a cardiac event such as an ST segment elevation that is indicative of an acute myocardial infarction.

A clock/timing sub-system 49 provides the means for timing specific activities of the cardiosaver system 5 including the absolute or relative time stamping of detected cardiac events. The clock/timing sub-system 49 can also facilitate power savings by causing components of the cardiosaver system 5 to go into a low power stand-by mode in between times for electrogram signal collection and processing. Such cycled power savings techniques are often used in implantable pacemakers and defibrillators. In an alternative embodiment, the clock/timing sub-system can be provided by a program subroutine run by the central processing unit 44. It is also envisioned that the processor 44 may include an integral or external First-In-First-Out (FIFO) buffer memory to allow the retention of data from before the detection of a cardiac event.

Techniques for detecting cardiac events by the processor 44 are described by Fischell et al in U.S. Patent No. 6,609,023.

An important aspect of the present invention is the filtering of the electrical signals sensed by the electrodes 4 and 8. The preferred embodiment of the present invention cardiosaver 11 will include high pass and/or low pass filtering of the electrical signals in the amplifier circuit 36. Alternative embodiments would introduce filtering in any one, two or all of the following locations:
1. a separate analog filter as part of the amplifier circuit 36 or between the amplifier circuit 36 and analog-to-digital converter 41,
2. a separate digital filter circuit placed within the analog-to-digital converter 41 or between the analog-to-digital converter 41 and the processor 44,
3. digital filtering performed by the processor 44 on the digital signals 38 and/or,
4. filtering performed by circuitry or software at any location within the implanted cardiosaver system.

The memory 47 includes specific memory locations for patient data, electrogram segment data and any other relevant data.

It is envisioned that the cardiosaver system 5 could also contain pacemaker circuitry 170 and/or defibrillator circuitry 180 similar to the cardiosaver system described by Fischell et al in U.S. Patent No. 6,272,379.

The alarm sub-system 48 contains the circuitry and transducers to produce the internal alarm signals for the cardiosaver 11. The internal alarm signal can be a mechanical vibration, a sound or a subcutaneous electrical tickle or shock.

The telemetry sub-system 46 with antenna 6 provides the cardiosaver system 5 with the means for two-way wireless communication to and from the external equipment 7 of FIG. 1. It is also envisioned that short-range telemetry such as that typically used in pacemakers and defibrillators could also be applied to the cardiosaver system 5. It is also envisioned that standard wireless protocols such as Bluetooth and 802.11a or 802.11b might be used to provide communication with a wider group of external devices.

A magnet sensor 190 can be incorporated into the cardiosaver system 5. The primary purpose for a magnet sensor 190 is to keep the cardiosaver system 5 in an off condition until it is checked out just before it is implanted into a patient. This can prevent depletion of the battery life in the period between the times that the cardiosaver system 5 is packaged at the factory until the day it is implanted. The present invention envisions the unique combination of an implanted cardiosaver system with a magnet sensor 190 to keep power turned off until immediately before or shortly after the device is implanted into a human subject.

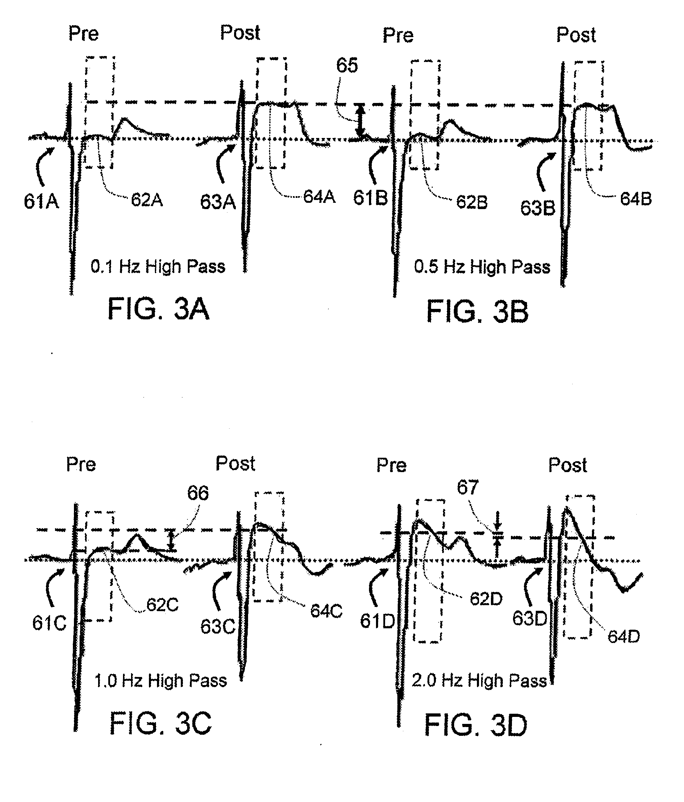

FIGS. 3A through 3D illustrate the effect of different high pass filter cut off frequency settings on the ability to detect ST segment shifts indicative of ST Elevation Acute Myocardial Infarction (STEMI). In FIGS. 3A through 3D, the ST segment shift is calculated as the average ST segment level where the ST segments are the portion of the beats that are enclosed within the dashed boxes of FIGS. 3A through 3D.

FIG. 3A shows a pre-occlusion heart beat electrogram 61A and post-occlusion heart beat electrogram 63A when filtered by a 0.1 Hz high pass filter. The pre-occlusion beat 61A has ST segment 62A and the post-occlusion beat 63A has a shifted ST segment 64A. The 0.1 Hz high pass filter used here has a voltage magnitude reduction of 6 dB at 0.05 Hz compared to the voltage magnitude at 0.15 Hz.

FIG. 3B shows a pre-occlusion heart beat electrogram 61B and post-occlusion heart beat electrogram 63B when filtered by a 0.5 Hz high pass filter. The pre-occlusion beat 61B has ST segment 62B and the post-occlusion beat 63B has an elevated ST segment 64B. The filter used here has a voltage magnitude reduction of 6 dB at 0.25 Hz compared to the voltage magnitude at 0.75 Hz. The ST segment voltage change (elevation) for both FIGS. 3A and 3B is illustrated by the length of the arrow 65 and is essentially the same for both cases. This is because neither the pre-occlusion ST segment 62B nor the post-occlusion ST segment 64B are significantly affected by the higher frequency filter used for the beats 61B and 63B.

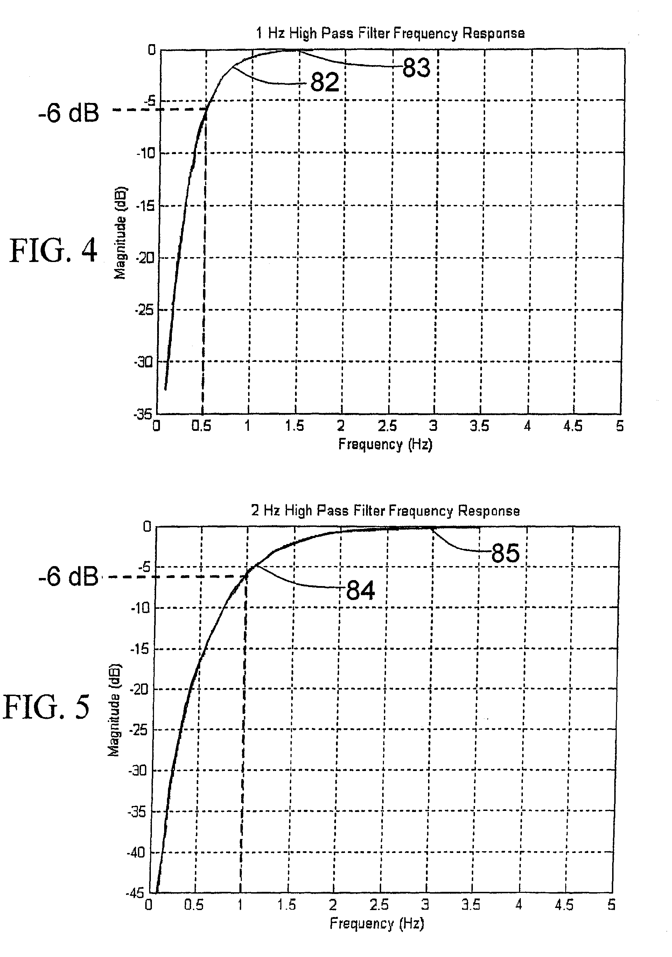

FIG. 3C shows a pre-occlusion heart beat electrogram 61C and post-occlusion heart beat electrogram 63C when filtered by a 1.0 Hz high pass filter. The pre-occlusion beat 61C has ST segment 62C and the post-occlusion beat 63C has an elevated ST segment 64C. The filter used here is shown in FIG. 4 and has a voltage magnitude reduction of 6 dB at 0.5 Hz compared to the signal at 1.5 Hz. The ST segment voltage change (elevation) for FIG. 3C is indicated by the length of the arrow 66. Although the length of the arrow 66 would be detectable, it is significantly less than the ST segment voltage change 65 seen in both FIG. 3A and FIG. 3B. This is caused by the excessive filtering effects on both the pre-occlusion ST segment 62C, which is pushed up, and the post-occlusion ST segment 64C, which is pushed down. Thus, even a 6 dB reduction at 0.5 Hz results in less accurate detection of ST segment elevation.

FIG. 3D shows a pre-occlusion heart beat electrogram 61D and post-occlusion heart beat electrogram 63D when filtered by a 2 Hz high pass filter. The pre-occlusion beat 61D has ST segment 62C and the post-occlusion beat 63C has a shifted ST segment 64C. The filter used here is shown in FIG. 5 and has a voltage magnitude reduction of 6dB compared to the signal at 3 Hz. The ST shift for FIG. 3D is indicated by the length of the arrow 67. While the shifts of FIGS. 3A, 3B and 3C clearly show ST elevation, the shift 67 of FIG. 3D is not only close to zero but is slightly negative. Therefore, detection of ST segment elevation is clearly undoable unless there is less than 6 dB of electrogram signal attenuation for frequencies at or above 1.0 Hz.

FIG. 4 shows the frequency response curve 82 of the 1 Hz high pass filter used to process the electrogram beats of FIG. 3C. The frequency response curve 82 has a magnitude fall off of 6 dB at 0.5 Hz compared to the magnitude of the signal at 1.5 Hz. It should be noted that for the filter of both FIG. 4, the signal has essentially no attenuation at three times the frequency at which there is 6 dB of attenuation. In other words, if there is 6 dB of attenuation at 0.5 Hz then the signal is essentially unattenuated at 1.5 Hz as shown by element 83.

FIG. 5 shows the frequency response curve 84 of the 2 Hz high pass filter used to process the electrogram beats of FIG. 3D. The frequency response curve 84 has a magnitude fall off of 6 dB at 1.0 Hz compared to the magnitude of the signal at 3.0 Hz. It should be noted that for the filter of FIG. 5, the signal has essentially no attenuation at three times the frequency at which there is 6 dB of attenuation. In other words, if there is 6 dB of attenuation at 1.0 Hz, then the signal is essentially unattenuated at 3.0 Hz as shown by element 85.

FIGS. 4 and 5 shows single pole filters that provides 6 dB of attenuation per octave. It is certainly envisioned that a two pole filter having essentially 12 dB of attenuation per octave or even an N- pole filter having N x 6 dB attenuation per octave could be used.

Thus it is clear from FIGS. 3A through 3D that a high pass filter with a voltage magnitude reduction of 6dB or greater at 1 Hz will attenuate too much of the low frequency information to accurately detect changes in ST segment voltage. Thus the present invention cardiosaver system must utilize a high pass filter having a voltage magnitude reduction that is less than 6 dB at 1 Hz. While the filter of FIG. 4 with a reduction of 6dB at 0.5 Hz is acceptable it would still be preferable to have still less attenuation than that shown in FIG. 4. Thus the preferred high pass filter should have a magnitude reduction of less than 6 dB at 0.5 Hz as compared to the electrogram signal voltage at 1.5 Hz.

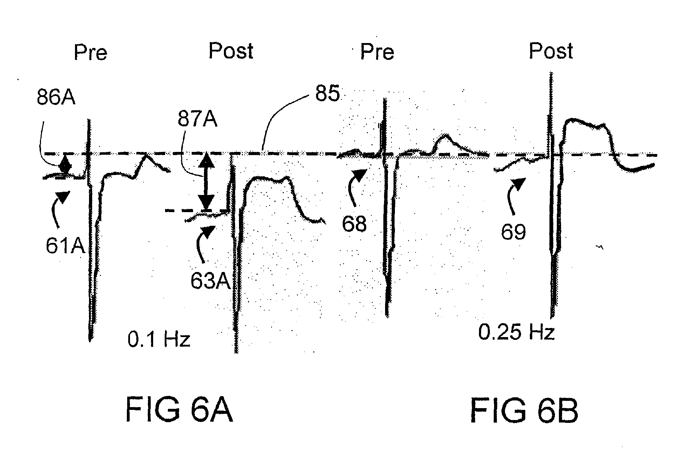

FIG. 6A shows the pre-occlusion heart beat electrogram 61A and post-occlusion heart beat electrogram 63A of FIG. 3A where the zero voltage level is marked by the dashed line 85. The beats 61A and 63A have been filtered by a 0.1 Hz high pass filter with a voltage magnitude reduction of 6dB at 0.05 Hz compared to the voltage magnitude at 0.15 Hz. The beats 61A and 63A show considerable d-c drift levels, the magnitude of the drift being illustrated by the length of the arrows 86A and 87A with respect to the zero voltage level 85. Thus, a 0.1 Hz high pass filter that would typically have 6 dB attenuation at 0.05 Hz and minimal attenuation at 0.1 Hz allows more d-c drift of the electrogram signal than is desirable for the accurate detection of ST segment deviations.

FIG. 6B shows the beats 68 and 69 where the same pre-occlusion and post-occlusion electrogram beats as shown for the beats 61A and 63A have now been processed with a 0.25 Hz high pass filter having a voltage magnitude reduction of 6 dB at 0.125 Hz compared to the signal magnitude at 0.375 Hz. As in FIG. 6A the zero voltage level is marked by the dashed line 85. It is clearly seen that the change in filtering from a 0.1 Hz high pass filter to a 0.25 Hz high pass filter essentially eliminates the d-c drift in beats 68 and 69. Removing d-c drift improves the effectiveness of an algorithm for the detection of ST shift. Although the Fischell et al algorithm of U.S. Patent No. 6,609,023 uses the ST segment minus the PQ segment to minimize the effects of drift, even the Fischell et al algorithm would be enhanced by using appropriate filtering to produce an electrogram signal with minimal d-c drift. Thus it is important to have a high pass filter with a voltage magnitude reduction that is greater than 6 dB at 0.05 Hz as compared with the signal at 0.15 Hz. The preferred embodiment of the presen0t invention has a high pass filter with a voltage magnitude reduction of greater than 6 dB at 0.1 Hz as compared to the signal at 0.3 Hz in order to limit the d-c drift.

The FIGS. 3A, 3B, 3C and 3D when considered together clearly indicate that the accurate detection of a shift in the voltage magnitude of the ST segment of the electrogram requires that the high pass filter produce less than 6 dB of attenuation for frequencies at and above 1.0 Hz and ideally at and above 0.5 Hz. This is quite different from conventional pacemakers and ICDs that typically have high pass filters that produce greater than 6 dB of attenuation at 0.5 Hz. Pacers and ICDs that attenuate the electrogram by more than 6 dB at frequencies below 1.0 Hz would not accurately detect shifts of the ST segment that are indicative of ischemia. It is clear from FIGS. 3A through 3D that a high pass filter with a voltage magnitude reduction of 6dB or greater at 1 Hz will attenuate too much of the low frequency information to allow accurate detection of changes in ST segment level. Thus the present invention cardiosaver system must utilize a high pass filter having a voltage magnitude reduction that is less than 6 dB at 1 Hz.

With the assistance of FIG. 6A and 6B, it has been shown that it is desirable to avoid d-c drift in the electrogram signal by setting the high pass filter to have greater than 6dB of attenuation at 0.05 Hz with the preferred high pass filter having greater than 6 dB of attenuation at 0.1 Hz. Thus, for ischemia detection with an implanted device, an acceptable range for the high pass filter should have less than 6 dB of electrogram signal voltage attenuation at and above 1.0 Hz compared to the signal magnitude at 3 Hz but more than 6 dB of attenuation at frequencies at and below 0.05 Hz. The preferred range for the frequency of the high pass filter would have less than 6 dB of electrogram voltage attenuation at and above 0.5 Hz compared to the electrogram signal voltage at 1.5 Hz but more than 6 dB of attenuation for frequencies at and below 0.1 Hz.

Similarly, since the ST segment is a relatively low frequency signal, there is no need to include signals at frequencies above 50 Hz. A low pass filter with a greater than 6 dB voltage magnitude reduction at 60 Hz will reduce interference from a-c power signals for any country using 60 Hz power generation. A low pass filter with a greater than 6 dB voltage magnitude reduction at 50 Hz will reduce interference from a-c power signals for any country using 50 Hz power generation. As the 50 Hz low pass filter would also be effective in countries with 60 Hz a-c power, it is the preferred embodiment of the present invention. The ideal low pass filter would have at least 6 dB of electrogram signal voltage attenuation at 60 or 50 Hz compared to the signal voltage at 25 Hz.

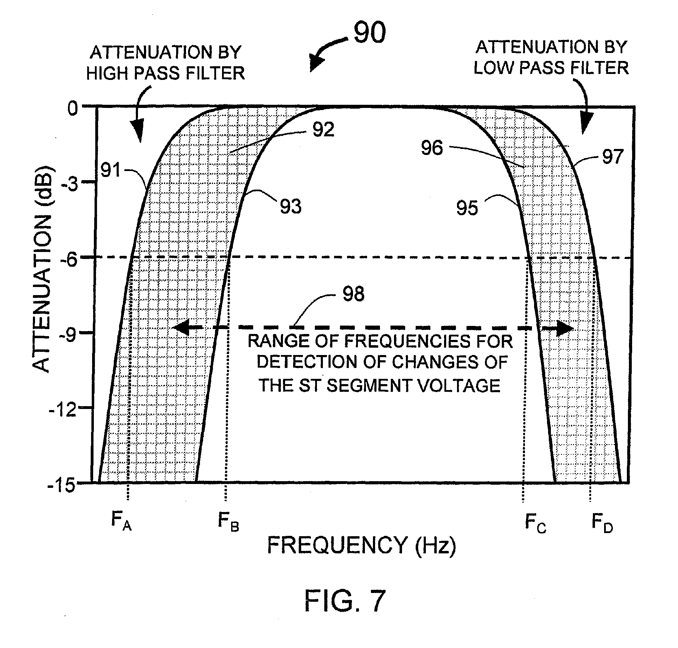

FIG. 7 illustrates some of the inventive concepts of the present invention. Specifically, FIG. 7 shows the attenuation of an electrogram signal by the filtering of the cardiosaver system 5 of FIGS. 1 and 2 as a function of frequency. The most important concept shown in FIG. 7 is that the high pass filter attenuation curve must lie within the band 92 bounded by the high pass filter curve 91 having 6 dB of attenuation at frequency F_{A} and the high pass filter curve 93 having 6 dB of attenuation at frequency F_{B}. For the present invention cardiosaver system with ST segment shift detection, it is highly desirable to have the high pass filter curve lie within the band 92 where the frequencies for the 6 dB attenuation points of the high pass filter curves 91 and 93 are between an F_{A} > 0.05 Hz and F_{B} < 1.0 Hz. The preferred embodiment of the present invention would utilize a filter curve that lies between the curves 91 and 93 where F_{A}> 0.1 Hz and F_{B} < 0.5 Hz.

FIG. 7 also shows a low pass filtering band 96 that is bounded by the low pass filter curves 95 and 97 with the 6 dB attenuation points at frequencies F_{C} and F_{D} respectively. One aspect of the present invention cardiosaver system 5 of FIGS. 1 and 2 is the use of a low pass filter that lies within the band 96. For countries using 60 Hz power (e.g. in the USA) it is only necessary that the upper bound curve 97 have a 6 dB attenuation point at F_{D} less than or equal to 60 Hz. Since Europe and many other countries use 50 Hz power, a preferred embodiment that would work in countries with either 50 or 60 Hz power, would have the upper bound curve 97 have a 6 dB attenuation point at F_{D} less than or equal to 50 Hz. The lower bound curve 95 cannot have F_{C} set too low or it will affect the ability to accurately track R waves or other high frequency components of the electrogram. The present invention would have F_{C} being equal to or greater than 25 Hz.

Although the low pass filtering and high pass filtering are independent of each other, the optimum filtering of the electrogram to obtain the best ST segment shift detection can use both high pass and low pass filtering as described with the assistance of FIG. 7.

Aspects of the invention are set out in the following series of numbered clauses.
1. An implanted system for the detection of coronary ischemia in a human patient, the system including:
   at least two electrodes for obtaining an electrical signal from the patient's heart, the electrical signal being an electrogram that has a signal voltage;
   a high pass filter designed to filter the electrical signal from the patient's heart, the high pass filter producing a reduction in the magnitude of the signal voltage that is less than 6 dB at 1Hz compared to the magnitude of the signal voltage at 3 Hz but more than 6 dB of attenuation at frequencies below 0.05 Hz, the output of the high pass filter being a filtered electrical signal; and,
   an electrical signal processor designed to process the filtered electrical signal from the high pass filter, the processor having the capability to detect a change in the ST segment signal voltage that is indicative of coronary ischemia.
2. The system of clause 1 where the high pass filter designed to filter the electrical signals from the patient's heart produces a reduction in the magnitude of the signal voltage that is less than 6 dB at 0.5 Hz compared to the magnitude of the signal voltage at 1.5 Hz but has more than 6 dB of attenuation at frequencies below 0.1 Hz
3. The system of clause 1 where the high pass filter is an N-pole filter where N is a number between 1 and 3.
4. The system of clause 1 further including a patient alerting capability designed to alert the patient following the detection of coronary ischemia.
5. The system of clause 4 where the patient alerting capability is from an internal alarm signal generated within the implanted system.
6. The system of clause 5 where the patient alerting capability includes a vibrational alarm signal.
7. The system of clause 5 where the patient alerting capability includes an acoustic alarm signal.
8. The system of clause 5 where the patient alerting capability includes an electrical tickle alarm signal.
9. The system of clause 4 further including an external alarm transceiver capable of wireless data communication with the implanted system where the patient alerting capability is from either or both the internal alarm an external alarm signal generated by the external alarm transceiver.
10. The system of clause 9 where the patient alerting capability includes an acoustic alarm signal.
11. The system of clause 9 where the patient alerting capability includes a vibrational alarm signal.
12. The system of clause 1 where the high pass filter is an analog filter.
13. They system of clause 1 where the high pass filter is a digital filter.
14. The system of clause 1 further including a low pass filter producing a greater than 6 dB reduction in the magnitude of the signal voltage at 60 Hz as compared to the magnitude of the signal voltage at 25 Hz.
15. The system of clause 1 further including a low pass filter producing a greater than 6 dB reduction in the magnitude of the signal voltage at 50 Hz as compared to the magnitude of the signal voltage at 25 Hz.
16. An implanted system for the detection of coronary ischemia in a human patient, the system including:
   at least two electrodes for obtaining an electrical signal from the patient's heart, the electrical signal being an electrogram that has a signal voltage;
   a low pass filter designed to filter the electrical signals from the patient's heart, the low pass filter producing a reduction in the magnitude of the signal voltage that is greater than 6 dB at 60 Hz compared to the magnitude of the signal voltage at 25 Hz, the output of the low pass filter being a filtered electrical signal; and,
   an electrical signal processor designed to process the filtered electrical signals from the low pass filter, the processor having the capability to detect a change in the ST segment signal voltage that is indicative of coronary ischemia.
17. The system of clause 16 where the low pass filter produces a reduction in the magnitude of the signal voltage that is greater than 6 dB at 50 Hz as compared to the magnitude of the signal voltage at 25 Hz.
18. The system of clause 16 where low pass filter is an N-pole filter where N is a number between 1 and 3.
19. The system of clause 16 further including a patient alerting capability designed to signal the patient following the detection of a cardiac event.
20. The system of clause 19 where the patient alerting capability is from an internal alarm signal generated within the implanted system.
21. The system of clause 20 where the patient alerting capability includes a vibrational alarm signal.
22. The system of clause 20 where the patient alerting capability includes an acoustic alarm signal.
23. The system of clause 20 where the patient alerting capability includes an electrical tickle alarm signal.
24. The system of clause 19 further including an external alarm transceiver capable of wireless data communication with the implanted system where the patient alerting capability is from an external alarm signal generated by the external alarm transceiver.
25. The system of clause 24 where the patient alerting capability includes an acoustic alarm signal.
26. The system of clause 24 where the patient alerting capability includes a vibrational alarm signal.
27. The system of clause 16 where the low pass filter is an analog filter.
28. They system of clause 16 where the low pass filter is a digital filter.
29. The system of clause 16 further including a high pass filter producing a less than 6 dB reduction in the magnitude of the signal voltage at 1.0 Hz as compared to the magnitude of the signal voltage at 3.0 Hz.
30. The system of clause 16 further including a high pass filter producing a greater than 6 dB reduction in the magnitude of the signal voltage at 0.1 Hz as compared to the magnitude of the signal voltage at 3.0 Hz.
31. An implanted cardiac pacemaker that includes the capability for detecting coronary ischemia, the pacemaker including:
   a lead designed to sense the electrical signal from the patient's heart, the electrical signal being an electrogram having a signal voltage;
   electronic circuitry to which the lead is electrically connected, the electronic circuitry being designed to pace the patient's heart; the electronic circuitry also including a high pass filter designed to filter the electrical signal from the patient's heart, the high pass filter producing a reduction in the magnitude of the signal voltage that is less than 6 dB at 1.0 Hz as compared to the magnitude of the electrical signal at 3.0 Hz but more than 6 dB of signal attenuation at frequencies below 0.05 Hz, the output of the high pass filter being a filtered electrical signal; and,
   an electrical signal processor designed to process the filtered electrical signal, the processor having the capability to detect a change in the ST segment signal voltage that is indicative of coronary ischemia.
32. The pacemaker of clause 31 where the high pass filter designed to filter the electrical signals from the patient's heart produces a reduction in the magnitude of the signal voltage that is less than 6 dB at 0.5Hz compared to the magnitude of the signal voltage at 1.5 Hz but has more than 6 dB of attenuation at frequencies below 0.1 Hz
33. The pacemaker of clause 31 where high pass filter is an N-pole filter where N is a number between 1 and 3.
34. The pacemaker of clause 31 further including a patient alerting capability designed to signal the patient following the detection of a cardiac event.
35. The pacemaker of clause 34 where the patient alerting capability is from an internal alarm signal generated within the implantable pacemaker.
36. The pacemaker of clause 35 where the patient alerting capability includes a vibrational alarm signal.
37. The pacemaker of clause 35 where the patient alerting capability includes an acoustic alarm signal.
38. The pacemaker of clause35 where the patient alerting capability includes an electrical tickle alarm signal.
39. The pacemaker of clause 34 further including an external alarm transceiver capable of wireless data communication with the implanted pacemaker where the patient alerting capability is from an external alarm signal generated by the external alarm transceiver.
40. The pacemaker of clause 39 where the patient alerting capability includes an acoustic alarm signal.
41. The pacemaker of clause 39 where the patient alerting capability includes a vibrational alarm signal.
42. The pacemaker of clause 31 where the high pass filter is an analog filter.
43. They pacemaker of clause 31 where the high pass filter is a digital filter.
44. The pacemaker of clause 31 further including a low pass filter producing a greater than 6 dB reduction in the magnitude of the signal voltage at 60 Hz as compared to the magnitude of the signal voltage at 25 Hz.
45. The pacemaker of clause 31 further including a low pass filter producing a greater than 6 dB reduction in the magnitude of the signal voltage at 50 Hz as compared to the magnitude of the signal voltage at 25 Hz.
46. An implantable cardiac defibrillator that includes the capability for ischemia detection, the defibrillator including:
   a lead designed to sense the electrical signal from a patient's heart; the electrical signal being an electrogram having a signal voltage;
   electronic circuitry to which the lead is electrically connected, the electronic circuitry being designed to defibrillate the patient's heart when ventricular fibrillation is sensed; the electronic circuitry also including a high pass filter designed to filter the electrical signal from the patient's heart, the high pass filter producing a reduction in the magnitude of the signal voltage that is less than 6 dB at 1.0 Hz as compared to the magnitude of the electrical signal at 3.0 Hz but more than 6 dB of signal attenuation at frequencies below 0.05 Hz, the output of the high pass filter being a filtered electrical signal; and,
   an electrical signal processor designed to process the filtered electrical signal, the processor having the capability to detect a change in the ST segment of the filtered electrical signal that is indicative of coronary ischemia.
47. The implantable cardiac defibrillator of clause 46 where the high pass filter designed to filter the electrical signals from the patient's heart produces a reduction in the magnitude of the signal voltage that is less than 6 dB at 0.5Hz compared to the magnitude of the signal voltage at 1.5 Hz but has more than 6 dB of attenuation at frequencies below 0.1 Hz.
48. The implantable cardiac defibrillator of clause 46 where high pass filter is an N-pole filter where N is a number between 1 and 3.
49. The implantable cardiac defibrillator of clause 46 further including a patient alerting capability designed to signal the patient following the detection of a cardiac event.
50. The implantable cardiac defibrillator of clause49 where the patient alerting capability is from an internal alarm signal generated within the implantable cardiac defibrillator.
51. The implantable cardiac defibrillator of clause 50 where the patient alerting capability includes a vibrational alarm signal.
52. The implantable cardiac defibrillator of clause 50 where the patient alerting capability includes an acoustic alarm signal.
53. The implantable cardiac defibrillator of clause 50 where the patient alerting capability includes an electrical tickle alarm signal.
54. The implantable cardiac defibrillator of clause 49 further including an external alarm transceiver capable of wireless data communication with the implantable cardiac defibrillator where the patient alerting capability is from an external alarm signal generated by the external alarm transceiver.
55. The implantable cardiac defibrillator of clause 54 where the patient alerting capability includes an acoustic alarm signal.
56. The implantable cardiac defibrillator of clause 54 where the patient alerting capability includes a vibrational alarm signal.
57. The implantable cardiac defibrillator of clause 46 where the high pass filter is an analog filter.
58. They implantable cardiac defibrillator of clause 46 where the high pass filter is a digital filter.
59. The implantable cardiac defibrillator of clause 46 further including a low pass filter producing a greater than 6 dB voltage magnitude reduction at 60 Hz.
60. The implantable cardiac defibrillator of clause 46 further including a low pass filter producing a greater than 6 dB voltage magnitude reduction at 50 Hz.
61. An implantable system for the detection of coronary ischemia in a human patient, the system including:
   at least two electrodes for obtaining an electrical signal from the patient's heart, the electrical signal being an electrogram that has a signal voltage;
   a high pass filter designed to filter the electrical signal from the patient's heart, the high pass filter producing an attenuation of the electrogram signal voltage by at least 6 dB at a frequency that is higher than 0.05 Hz but lower than 1.0 Hz compared to the signal voltage at 3.0 Hz but less than 6 dB of signal voltage attenuation at all frequencies higher than 1.0 Hz and lower than 3.0 Hz compared to the signal voltage at 3.0 Hz; and,
   an electrical signal processor designed to process the filtered electrical signal from the high pass filter, the processor having the capability to detect a change in the ST segment signal voltage that is indicative of coronary ischemia.
62. An implanted system for the detection of coronary ischemia in a human patient, the system including:
   at least two electrodes for obtaining an electrical signal from the patient's heart, the electrical signal being an electrogram that has a signal voltage;
   a high pass filter designed to filter the electrical signal from the patient's heart, the high pass filter producing an attenuation of the electrogram signal voltage by at least 6 dB at a frequency that is higher than 0.1 Hz but lower than 0.5 Hz compared to the signal voltage at 1.5 Hz but less than 6 dB of signal voltage attenuation at all frequencies higher than 0.5 Hz and lower than 1.5 Hz compared to the signal voltage at 1.5 Hz; and,
   an electrical signal processor designed to process the filtered electrical signal from the high pass filter, the processor having the capability to detect a change in the ST segment signal voltage that is indicative of coronary ischemia.
63. An implanted system for the detection of coronary ischemia in a human patient, the system including:
   at least two electrodes for obtaining an electrical signal from the patient's heart, the electrical signal being an electrogram that has a signal voltage, the electrodes being direct coupled to an amplifier that is part of the implanted system so that there is no high pass filtering of the electrogram signal voltage, the amplifier having an output signal free from high pass filtering, and
   an electrical signal processor designed to process the output signal from the amplifier, the processor further having the capability to detect coronary ischemia by measurements of a change in the difference between the electrogram voltage of the ST segment and the electrogram voltage of the PQ segment, the voltage difference being the ST deviation which is an indication of coronary ischemia.
64. The implanted system of clause 63 further including a low pass filter that provides at least 6 dB of electrogram signal voltage attenuation at a frequency of 60 Hz compared to the signal voltage at 25 Hz.

Various other modifications, adaptations, and alternative designs are of course possible in light of the above teachings. Therefore, it should be understood at this time that, within the scope of the appended claims, the invention can be practiced otherwise than as specifically described herein.

## Claims

1. An implanted system for the detection of coronary ischemia in a human patient, the system including:
at least two electrodes for obtaining an electrical signal from the patient's heart, the electrical signal being an electrogram that has a signal voltage;
a high pass filter designed to filter the electrical signal from the patient's heart, the high pass filter producing a reduction in the magnitude of the signal voltage that is less than 6dB at 1Hz compared to the magnitude of the signal voltage at 3 Hz but more than 6 dB of attenuation at frequencies below 0.05 Hz, the output of the high pass filter being a filtered electrical signal; and
an electrical signal processor designed to process the filtered electrical signal from the high pass filter, the processor having the capability to detect a change in the ST segment signal voltage that is indicative of coronary ischemia.

2. The system of claim 1 where the high pass filter designed to filter the electrical signals from the patient's heart produces a reduction in the magnitude of the signal voltage that is less than 6 dB at 0.5 Hz compared to the magnitude of the signal voltage at 1.5 Hz but has more than 6 dB of attentuation at frequencies below 0.1 Hz.

3. The system of claim 1 or claim 2 where the high pass filter is an N-pole filter where N is a number between 1 and 3.

4. The system of any preceding claim further including a patient alerting capability designed to alert the patient following the detection of coronary ischemia.

5. The system of claim 4 where the patient alerting capability is from an internal alarm signal generated within implanted system.

6. The system of claim 4 or claim 5 where the patient alerting capability includes a vibrational alarm system.

7. The system of any of claims 4 to 6 where the patient alerting capability includes an acoustic alarm signal.

8. The system of any of claims 4 to 7 where the patient alerting capability includes an electrical tickle alarm signal.

9. The system of any of claims 4 to 8 further including an external alarm transceiver capable of wireless data communication with the implanted system where the patient alerting capability is from either of both the internal alarm an external alarm signal generated by the external alarm transceiver.

10. The system of any preceding claim where the high pass filter is an analog filter.

11. The system of any of claims 1 to 9 where the high pass filter is a digital filter.

12. The system of any preceding claim further including a low pass filter producing a greater than 6 dB reduction in the magnitude of the signal voltage at 60 Hz as compared to the magnitude of the signal voltage at 25 Hz.

13. The system of any preceding claim further including a low pass filter producing a greater than 6 dB reduction in the magnitude of the signal voltage at 50 Hz as compared to the magnitude of the signal voltage at 25 Hz.
